# EUROPEAN PATENT APPLICATION

(11) **EP 3 646 920 A1**
(43) Date of publication of application: **06.05.2020**
(21) Application number: 18208686.8
(22) Date of filing: 27.11.2018
(51) Int. Cl.: A61N 1/36, A61F 7/00, A61N 2/00, A61N 5/06

(54) **WIRELESS PHYSICAL STIMULATION SYSTEM AND METHOD**

(30) Priority: 29.10.2018 TW 107138192
(71) Applicant: Institute for Information Industry, Taipei (TW)
(72) Inventor: Liu, Chih-Yun, Taipei Cninese Taipei (TW); Chen, An-Chun, 111 Taipei City (TW); Chang, Ya-Chi, 510 Yuanlin City, Changhua County (TW); Wei, Shih-Yao, 116 Taipei City (TW); Lin, Tse-Yu, 220 New Taipei City (TW)
(74) Representative: Michalski Hüttermann & Partner Patentanwälte mbB

(57) **Abstract**

A wireless physical stimulation system includes at least one sensor, at least one physical stimulation device and an electronic device. The electronic device wirelessly connects to the at least one sensor disposed on a human body and the at least one physical stimulation device disposed on the human body. The at least one sensor is configured to sense at least one first sensing signal. The electronic device is configured to receive the least one sensing signal from the least one sensor; generate first body condition information on the basis of a body condition identifying model and according to the least one sensing signal; generate first feedback plan information on the basis of a first feedback model and according to the first body information; and control the at least one physical stimulation device according to the first feedback plan information.

## Description

### CROSS-REFERENCES TO RELATED APPLICATIONS

Not applicable.

### BACKGROUND OF THE INVENTION

### Field of the Invention

Embodiments of the present invention relates to a wireless physical stimulation system and method. More specifically, the embodiments of the present invention relate to a system and method to perform wireless physical stimulation based on analyzing physiological signals with a physiological signal model.

### Descriptions of the Related Art

Conventional devices such as electric stimulation devices and electric heated blankets are mostly wired devices. Further, the intensity of thermal stimulation or the intensity of electric stimulation is based on the operator's experience to operate the device directly, but it usually cannot provide the most suitable feedback plan to the body. Further, the conventional devices are not equipped with the functions of monitoring the physiologic signals, cannot record physiologic data and analyze physiologic signals, and cannot provide suitable feedback plans for different fatigue conditions to ease a user's fatigue more efficiently.

Accordingly, a common effort shall be made in the field to provide a more efficient wireless physical stimulation system and method.

### SUMMARY OF THE INVENTION

In order to achieve the aforesaid purposes, the embodiments of the present invention provide a wireless physical stimulation system. The wireless physical stimulation system comprises at least one sensor, at least one physical stimulation device, and an electronic device, wherein the at least one sensor and the at least one physical stimulation device are disposed on a human body. The at least one sensor is configured to detect at least one first sensing signal. The electronic device comprises a transceiver and a processor, which are electrically connected to each other. The transceiver is configured to wirelessly connect to the at least one sensor and the at least one physical stimulation device and receive the at least one first sensing signal from the at least one sensor. The processor is configured to generate first body condition information on the basis of a body condition identifying model and according to the least one first sensing signal; generate first feedback plan information on the basis of the first feedback model according to the first body condition information; and control the at least one physical stimulation device according to the first feedback plan information.

The embodiments of the present invention further provide a wireless physical stimulation system. The wireless physical stimulation system comprises at least one sensor, at least one physical stimulation device, and an electronic device, wherein the at least one sensor and the at least one physical stimulation device are disposed on a human body. The at least one sensor is configured to detect at least one first sensing signal. The electronic device comprises a transceiver. The transceiver is configured to connect to a cloud computing system, the at least one sensor and the at least one physical stimulation device; receive the at least one first sensing signal from the at least one sensor; transmit the at least one first sensing signal to the cloud computing system so that the cloud computing system generates first body condition information on the basis of a body condition identifying model and according to the at least one first sensing signal and generates first feedback plan information on the basis of a feedback model according to the first body condition information; receive the first feedback plan information from the cloud computing system; and transmit the first feedback plan information to the at least one physical stimulation device.

The embodiments of the present invention further provide a wireless physical stimulation method. The wireless physical stimulation method is adapted to a wireless physical stimulation system. The wireless physical stimulation system comprises at least one sensor, at least one physical stimulation device and an electronic device. The at least one sensor and the at least one physical device are disposed on a human body. The electronic device is wirelessly connected to the at least one sensor and the at least one physical stimulation device. The wireless physical stimulation method comprises the following steps of: detecting, by the at least one sensor, at least one first sensing signal; receiving, by the electronic device, the at least one first sensing signal from the at least one sensor; generating, by the electronic device, first body condition information on the basis of a body condition identifying model and according to the at least one sensing signal; generating, by the electronic device, first feedback plan information on the basis of a first feedback model and according to the first body condition information; and controlling, by the electronic device, at least one physical stimulation device according to the first feedback plan information.

The detailed technology and preferred embodiments implemented of the present invention are described in the following paragraphs accompanying the appended drawings for people skilled in this field to well appreciate the features of the claimed invention.

### BRIEF DESCRIPTION OF THE DRAWINGS

**FIG. 1** is a schematic view of a wireless physical stimulation system of the first embodiment of the present invention;
**FIG. 2** is a schematic view of a wireless physical stimulation system of the second embodiment of the present invention;
**FIG. 3** is a schematic view of a wireless physical stimulation system of the third embodiment of the present invention;
**FIG. 4** is a schematic view of a wireless physical stimulation system of the fourth embodiment of the present invention;
**FIG. 5A** is a schematic view of a wireless physical stimulation system of the fifth embodiment of the present invention;
**FIG. 5B** is an electromyogram measurement figure of the fifth embodiment of the present invention;
**FIG. 5C** is a schematic view of electromyogram frequency versus electromyogram intensity of the fifth embodiment of the present invention;
**FIG. 6** is a schematic view of a wireless physical stimulation system of the sixth embodiment of the present invention;
**FIG. 7** is a schematic view of a wireless physical stimulation system of the seventh embodiment of the present invention;
**FIG. 8** is a flow chart of a wireless physical stimulation method of the eighth embodiment of the present invention;
**FIG. 9** is a flow chart of a wireless physical stimulation method of the ninth embodiment of the present invention;
**FIG. 10** is a flow chart of a wireless physical stimulation method of the tenth embodiment of the present invention; and
**FIG. 11** is a flow chart of a wireless physical stimulation method of the eleventh embodiment of the present invention.

### DESCRIPTION OF THE PREFERRED EMBODIMENT

In the following description, the present invention will be explained with reference to embodiments thereof. It shall be noted that these embodiments are not intended to limit the present invention to any specific environment, applications or particular implementations described in these embodiments. Therefore, description of these embodiments is only for purpose of illustration rather than to limit the present invention, and the scope of this application shall be governed by the claims. It should be appreciated that, in the following embodiments and the attached drawings, elements unrelated to the present invention are omitted from depiction; and dimensional relationships among individual elements in the attached drawings are provided only for ease of understanding, but not to limit the actual scale. Except for the particular explanation, identical (or similar) elements may correspond to the identical (or similar) elements. Furthermore, terms "first", "second" and "third" used in the present specification are used to distinguish the order relation between the identical objects.

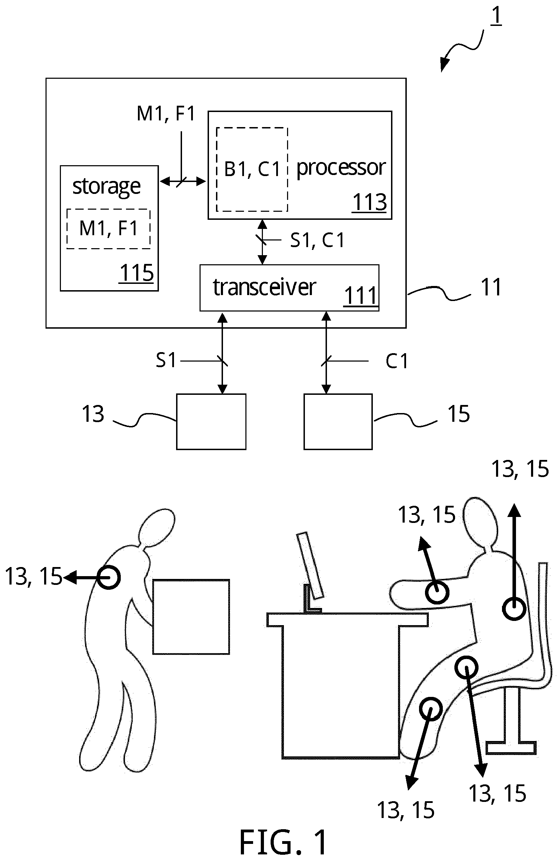

**FIG. 1** is a schematic view of a wireless physical stimulation system **1** of the first embodiment of the present invention. The content shown in the **FIG.1** is merely used to describe the embodiment of the present invention rather than limit the present invention. Please refer to **FIG.1**, the wireless physical stimulation system **1** basically comprises an electronic device **11**, at least one sensor **13**, and at least one physical stimulation device **15**. The electronic device **11** comprises a transceiver **111**, a processor **113** and a storage **115**, wherein the processor **113** is electrically connected to the transceiver **111** and the storage **115**.

The at least one sensor **13** and the at least one physical stimulation device **15** are disposed on a human body and are wirelessly connected to the transceiver **111**. For example, as shown in **FIG. 1**, a sensor **13** and a physical stimulation device **15** may be disposed on the back of the person on the left, or multiple sensors **13** and multiple physical stimulation devices **15** may be disposed on the thigh, calf, arm, and waist of the person on the right. It shall be noted that the content of the embodiment is not used to limit the number and positions of the sensors **13** and the physical stimulation devices **15** of the present invention.

Furthermore, each circle in **FIG. 1** comprises the at least one sensor **13** and the at least one physical stimulation device **15**. The at least one sensor **13** and the at least one physical stimulation device **15** may be arranged next to each other, be integrated in an element, or be independent elements. Each sensor **13** is configured to sense a sensing signal **S1**.

The processor **113** receives the at least one sensing signal **S1** from the at least one sensor **13** via transceiver **111**. The processor **113** is configured to generate first body condition information **B1** on the basis of a body condition identifying model **M1** and according to the least one first sensing signal **S1**. Then, the processor **113** is configured to generate first feedback plan information **C1** on the basis of a first feedback model **F1** and according to the first body condition information **B1**. Next, the processor **113** is configured to control the at least one physical stimulation device **15** according to the first feedback plan information **C1** to generate the corresponding physical stimulation.

It shall be noted that the electronic device **11** may be electronic product, such as a mobile device, a smart device, or a tablet. The transceiver **111** may comprise various internal connection interfaces (e.g., flat cables of various functions) so that multiple elements disposed in a same apparatus connect and transmit data with each other. In some embodiments, the transceiver **111** may also comprise various input/output interfaces so that multiple elements disposed in different apparatuses connect and transmit data with each other. The input/output interfaces may comprise various wireless communication interfaces (which are for example but not limited to: a Bluetooth interface, a Wi-Fi interface, a mobile communication network interface or the like).

Moreover, both of the sensor **13** and the physical stimulation device **15** comprise microcontroller and transceiver to transmit, receive and process relative signals and commands. The sensor **13** and the physical stimulation device **15** are equipped with independent power source for wireless operation.

In one or some implementations, the physical stimulation device **15** may be one or both of a wireless thermotherapy device and a wireless electrotherapy device. In some implementations, the physical stimulation device **15** may be a low energy laser light emitting diode (LED) radiating device or a magnetic field excitation device. The processor **113** controls one of the wireless thermotherapy device and the wireless electrotherapy device according to the first feedback plan information **C1**. In addition, in one or some implementations, the first feedback plan information **C1** may include a thermal stimulation plan and an electric stimulation plan.

It shall be noted that, the first feedback plan information **C1** includes control parameters which the electronic device **11** transmits to the physical stimulation device **15**. For example, if the physical stimulation device **15** is a thermal stimulation device, the control parameters may include duration time, temperature, etc. For another example, if the physical stimulation device **15** is an electric stimulation device, the control parameters may include duration time, intensity, frequency, waveform, etc. However, the present invention is not limited by the aforesaid examples.

In one or some implementations, the body condition identifying model **M1** and the first feedback model **F1** are stored in the storage **115** in advance. The processor **113** may access the data stored by the storage **115**. The establishment of the body condition identifying model **M1** may be based on measurement data, laboratory data, etc. The establishment of the first feedback model **F1** may be based on a fatigue position, a fatigue level, a type of physical stimulation, etc. In some implementations, the first feedback model may be established by an electronic apparatus comprising an input unit, an output unit, a memory unit, a logic processing and controlling unit. The input unit may receive sensing signals, and the output unit may generate different physical stimulation modes. The memory unit may memorize the logic processing results which are set by a user and used in the last time. The logic processing and controlling unit calculates, according to the changes of input signals and the data stored by the memory, a new physical stimulation method to control the output unit.

In one or some implementations, the electronic device **11** further comprises an input unit (not shown), which is electrically connected to the processor **113**. The input unit is configured to receive and transmit a user command to the processor **113**. Then, the processor **113** adjusts the physical stimulation device **15** according to the user command. In other words, a user may actively adjust the plan and intensity of the physical stimulation via the input unit.

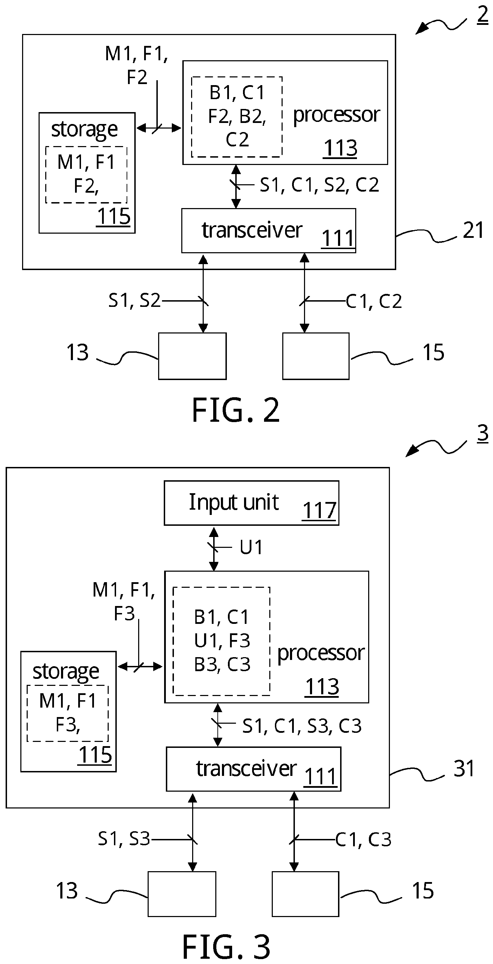

**FIG. 2** is a schematic view of a wireless physical stimulation system **2** of the second embodiment of the present invention. The wireless physical stimulation system **2** of the second embodiment is an extension of the wireless physical stimulation system **1** of the first embodiment. The elements with the identical symbols have similar functions; thus, the identical content is not repeatedly described. The differences are detailed merely in the following.

In the second embodiment, after the processor **113** transmits the first feedback plan information **C1** to the physical stimulation device **15**, the at least one sensor **13** further detects at least one second sensing signal **S2**. The processor **113** is further configured to receive the at least one second sensing signal **S2** from the at least one sensor **13**. In other words, after the electronic device **21** executes the physical stimulation, the electronic device **21** detects the reaction condition of the body muscle in response to the physical stimulation.

Afterwards, the processor **113** is further configured to establish a second feedback model **F2** according to the at least one second sensing signal **S2** and the first feedback model **F1**. The processor **113** is further configured to generate second body condition information **B2** on basis of the body condition identifying model **M1** and according to the least one second sensing signal **S2**. Then, the processor **113** is further configured to generate second feedback plan information **C2** on the basis of the second feedback model **F2** and according to the second body condition information **B2**. Then, the processor **113** is further configured to control the at least one physical stimulation device **15** according to the second feedback plan information **C2**.

In other words, in the second embodiment, after the wireless physical stimulation system **2** performs the physical stimulation, the wireless physical stimulation system **2** further uses the second sensing **S2** to adjust the first feedback model **F1** into the second feedback model **F2** as the use of physical stimulation plan for a specific user. Therefore, wireless physical stimulation system **2** may generate the second body condition information **C2** according to the modified the second feedback model **F2** to provide more effective physical stimulation.

In one or some implementations, the processor **113** may stores the second feedback model **F2** in the storage **115** so that the processor **113** further uses or modifies it later. Moreover, in one or some implementations, the second sensing signal **S2** includes an electromyogram (EMG) signal.

**FIG. 3** is a schematic view of a wireless physical stimulation system **3** of the third embodiment of the present invention. The wireless physical stimulation system **3** of the second embodiment is an extension of the wireless physical stimulation system **1** of the first embodiment. The elements with the identical symbols have similar functions; thus, the identical content is not repeatedly described. The differences are detailed merely in the following.

In the third embodiment, the electronic device **31** further comprises an input unit **117**, which is electrically connected to the processor **113**. The input unit **117** is configured to receive a user command **U1** inputted by a user. The processor **113** is further configured to establish a third feedback model **F3** according to the user command **U1** and the first feedback model **F1**. In other words, the user can adjust the first feedback model **F1** according to the feelings in response to the physical stimulation.

On the other hand, the at least one sensor **13** is further configured to detect at least one third sensing signal **S3** and transmit the at least one third sensing signal **S3** to the processor **113** of the electronic device **31**. The processor **113** is further configured to generate third body condition information **B3** on the basis of the body condition identifying model **M1** and according to the at least one third sensing signal **S3**. Next, the processor **113** is further configured to generate third feedback plan information **C3** on the basis of the third feedback model **F3** and according to the third body condition information **B3**. Then, the processor **113** is further configured to control the at least one physical stimulation device **15** according to the third feedback plan information **C3**.

In other words, in the third embodiment, after the wireless physical stimulation system **3** controls the physical stimulation device **15** according to the third feedback plan information **C3** to perform the physical stimulation to a user, the user may further use the user command **U1** and the first feedback model **F1** to establish the third feedback model **F3** which satisfies personal demand, for example, recording the best physical stimulation control plan used by a user the most often and performing customized adjustments according to the user command. Therefore, the wireless physical stimulation system **3** may generate the third feedback plan information **C3** according to the third feedback model **F3** to provide the physical stimulation which matches the user's expectation more.

In one or some implementations, processor **113** may stores the third feedback model **F3** in the storage **115** so that the processor **113** further uses or modifies it later. Moreover, in one or some implementations, the third sensing signal **S3** includes an electromyogram (EMG) signal.

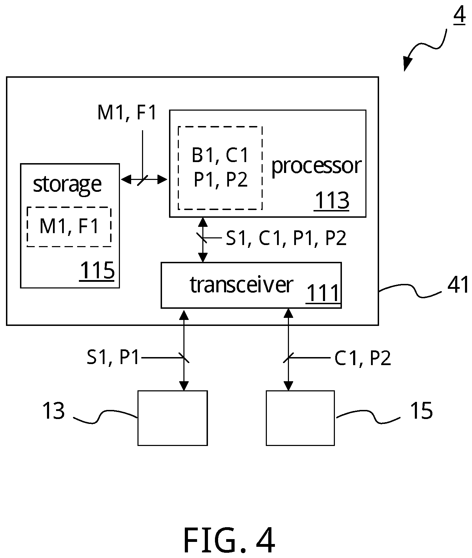

**FIG. 4** is a schematic view of a wireless physical stimulation system **4** of the fourth embodiment of the present invention. The wireless physical stimulation system **4** of the fourth embodiment is an extension of the wireless physical stimulation system **1** of the first embodiment. The elements with the identical symbols have similar functions; thus, the identical content is not repeatedly described. The differences are detailed merely in the following.

Specifically, in the fourth embodiment, the processor **113** is further configure to generate to generate a detection period signal **PI** and a feedback period signal **P2**, wherein the detection period signal **PI** and the feedback period signal **P2** do not overlap in the operation period with each other. The processor **113** transmits the detection period signal **PI** via the transceiver **111** to the at least one sensor **13** to control the detection period of the at least one sensor **13**. On the other hand, processor **113** transmits the feedback period signal **P2** via the transceiver **111** to the at least one physical stimulation device **15** to control the feedback period of the at least one physical stimulation device **15**. In other words, the wireless physical stimulation system **4** separates the operation durations of the sensor and the physical stimulation device to avoid measuring the incorrect measurement signals.

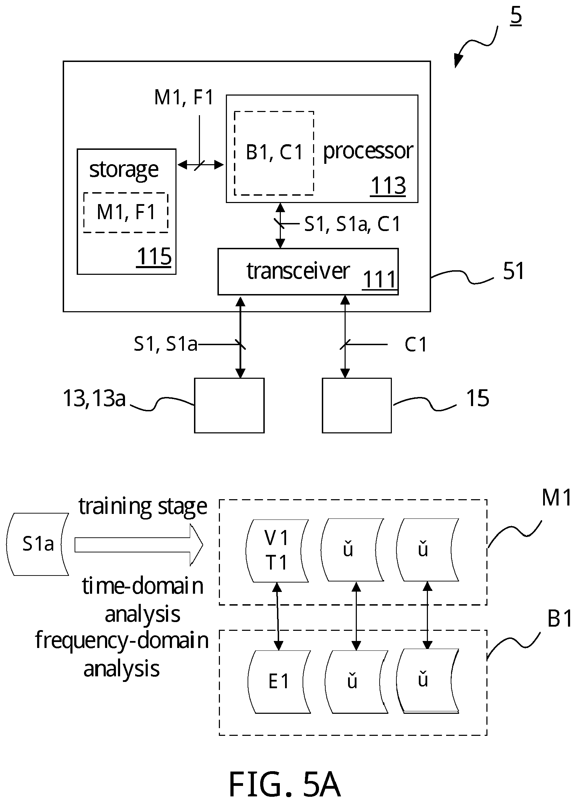

**FIG. 5A** is a schematic view of a wireless physical stimulation system **5** of the fifth embodiment of the present invention. The wireless physical stimulation system **5** of the fifth embodiment is an extension of the wireless physical stimulation system **1** of the first embodiment. The elements with the identical symbols have similar functions; thus, the identical content is not repeatedly described. The differences are detailed merely in the following.

Specifically, in the fifth embodiment, the at least one sensor **13** includes an electromyogram (EMG) sensor **13a**, and the electromyogram sensor **13a** is configured to detect an electromyogram signal **S1a**. Therefore, the at least one sensing signal **S1** includes at least one electromyogram signal **S1a**.

The body condition identifying model **M1** further comprises a fatigue characteristic curve **VI** and a characteristic threshold **T1**. The fatigue characteristic curve **VI** is established by its characteristic data obtained by time-domain analysis and frequency-domain analysis to the at least one electromyogram signal **S1a** at a data training stage by the wireless physical stimulation system **5**. The wireless physical stimulation system 5 further determines the characteristic threshold **T1** according to the characteristic data.

On the other hand, each of muscle fatigue condition information **E1** is recorded in the first body condition information **B1**. Each set of the fatigue characteristic curves **VI** and the characteristic threshold **T1** corresponds to one the muscle fatigue condition information **E1**. Therefore, the processor **113** is further configured to generate the muscle fatigue condition information **E1** of the first body condition information **B1** on the basis of the fatigue characteristic curve **VI** and the characteristic threshold **T1** of the body condition identifying model **M1** and according to the at least one EMG signal **S1a**. In other words, in the fifth embodiment, the body condition identifying model **M1** is further configured to record EMG signals of different muscle fatigue conditions, and the muscle fatigue conditions corresponding to the EMG signals are recorded in the first body condition information **B1**.

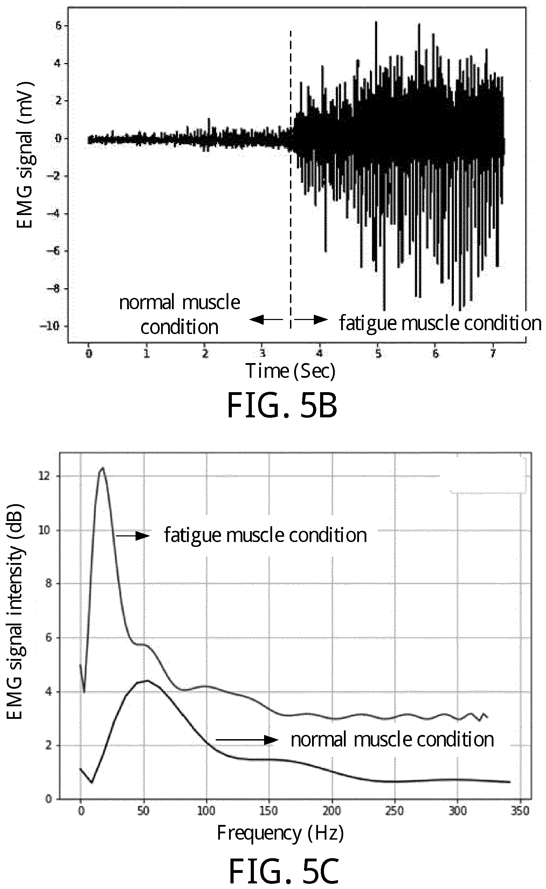

Please refer to **FIG. 5B** and **FIG. 5C** for an example. **FIG. 5B** depicts an electromyogram measurement figure. **FIG. 5C** depicts a schematic view of electromyogram frequency versus electromyogram intensity. In order to establish body condition identifying model, the electronic device **11** analyzes EMG signals of normal muscle and fatigue muscle (shown in **FIG. 5B**) of a particular human part at a data training stage. Next, the electronic device **11** analyzes the EMG signals in a time-domain and a frequency-domain to establish the corresponding characteristic curve (shown in **FIG. 5C**). Since the normal muscle EMG signal and the fatigue muscle signal are different in EMG frequency characteristic and EMG intensity characteristic, the electronic device **11** may determine the characteristic threshold of the corresponding characteristic curve. In other words, different types of muscle fatigue conditions have different characteristic curves, and the electronic device **11** may further record the muscle fatigue condition information in the body condition information.

It shall be noted that the aforesaid content is merely used for the explanation of establishing body condition identifying model according to the fatigue characteristic curve and the characteristic threshold and the explanation of generating muscle fatigue condition information according to the fatigue characteristic curve and the characteristic threshold, but it is not used to limit the present invention. As to different types of muscle fatigue conditions, different characteristics may be determined according to EMG signals and be set as characteristic thresholds, and people skilled in the art may achieve or change the features according to the aforesaid content; therefore, here is no need of further description.

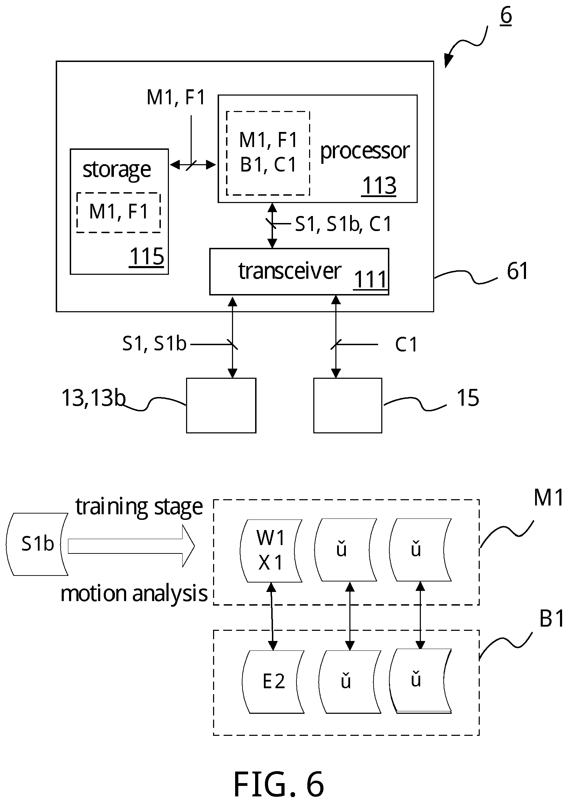

**FIG. 6** is a schematic view of a wireless physical stimulation system **6** of the sixth embodiment of the present invention. The wireless physical stimulation system **6** of the second embodiment is an extension of the wireless physical stimulation system **1** of the first embodiment. The elements with the identical symbols have similar functions; thus, the identical content is not repeatedly described. The differences are detailed merely in the following.

Specifically, the sensor **13** comprises a motion sensor **13b**, and the motion sensor **13b** is configured to detect a motion signal **S1b**. Therefore, the at least one sensing signal **S1** includes at least one motion signal **S1b**.

The body condition identifying model **M1** further comprises a motion posture template **W1** and a characteristic threshold **X1**. The motion posture template **W1** is established by its characteristic data obtained by analyzing the at least one motion signal **S1b** at a data training stage by the wireless physical stimulation system **6**. The wireless physical stimulation system **6** further determines the characteristic threshold **X1** according to the characteristic data.

On the other hand, each muscle fatigue condition information **E2** is recorded in the first body condition information **B1**. Each set of the motion posture template **W1** and the characteristic threshold **X1** corresponds to the muscle fatigue condition information **E2**. In other words, in the sixth embodiment, the body condition identifying model **M1** is further configured to record different motion signals corresponding to different muscle fatigue conditions, and the muscle fatigue condition information **E2** corresponding to the muscle fatigue conditions are recorded in the first body condition information **B1**.

For example, at a data training stage, electronic device **11** may record a specific motion according to a motion signal, for example, lifting a stuff, bending, or crouching, to establish the motion posture template. The electronic device **11** may record how many times, duration, frequency, amplitude, etc., to determine the characteristic threshold. Since different muscle fatigues corresponds to different motions, the electronic **11** may establish the relation therebetween. Therefore, the electronic device **11** may determine the muscle fatigue condition information of the first body condition information by analyzing motions and the characteristics of motions.

It shall be noted that the aforesaid content is merely used for the explanation of establishing body condition identifying model according to the motion posture template and the characteristic threshold and the explanation of generating muscle fatigue condition information according to the motion posture template and the characteristic threshold, but it is not used to limit the present invention. As to different types of muscle fatigue conditions, different characteristics may be determined according to motion signals and be set as characteristic thresholds, and people skilled in the art may achieve or change the features according to the aforesaid content.

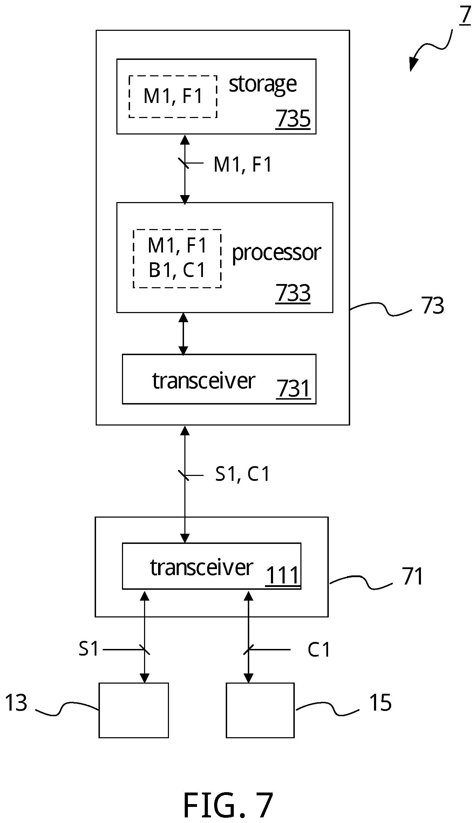

**FIG. 7** is a schematic view of a wireless physical stimulation system **7** of the seventh embodiment of the present invention. The wireless physical stimulation system **7** of the second embodiment is an extension of the wireless physical stimulation system **1** of the first embodiment. The elements with the identical symbols have similar functions; thus, the identical content is not repeatedly described. The differences are detailed merely in the following.

In the seventh embodiment, the transceiver **111** of the electronic device **71** is connected to a cloud computing system **73**. More specifically, the functions of processor **113** and the storage **115** are replaced with the cloud computing system **73**. The cloud computing system is equipped with a transceiver **731**, a processor **733**, and a storage **735**. The storage **735** is configure to store the body condition identifying model **M1** and first feedback model **F1**.

More specifically, after the at least one sensor **13** detects and transmits at least one first sensing signal **S1**, the electronic device **11** transmits the at least one first sensing signal **S1** via the transceiver **111** to the cloud computing system **73** so that the cloud computing system **73** generates first body condition information **B1** on the basis of the body condition identifying model **M1** and according to the at least one first sensing signal **S1** and generates first feedback plan information **C1** on the basis of a first feedback model **F1** and according to the first body condition information **B1**. Afterwards, the electronic device **71** receives the first feedback plan information **C1** from the cloud computing system **73** and transmits the first feedback plan information **C1** to the at least one physical stimulation device **15** to control the at least one physical stimulation device **15**.

In other words, in the wireless physical stimulation system **7**, the electronic device **11** is merely used to transmit the relevant measurement signals, control signals, etc. The operations of the at least one sensor **13** and the at least one physical stimulation device **15** is controlled by the cloud computing system **73**.

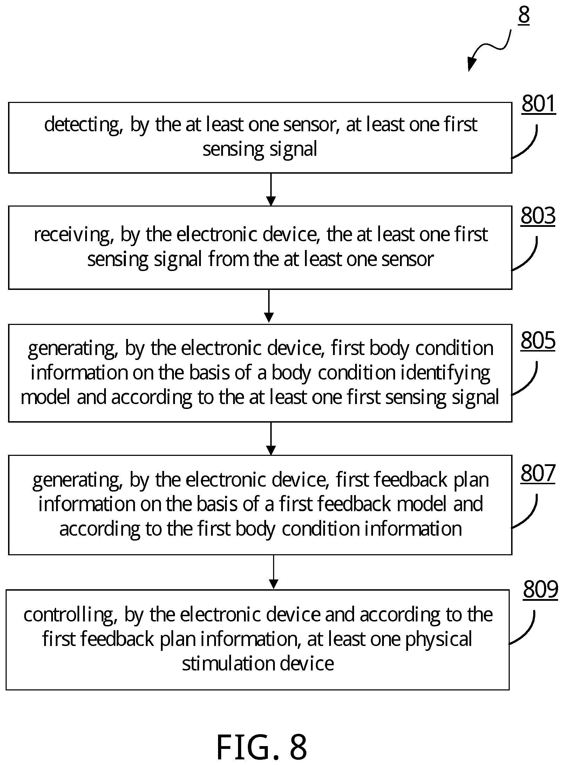

**FIG. 8** is a flow chart of a wireless physical stimulation method **8** of the eighth embodiment of the present invention. The content shown in **FIG. 8** is merely used to describe the embodiment of the present invention rather than limit the present invention. The wireless physical stimulation method **8** is adapted for a wireless physical stimulation system (e.g., the wireless physical stimulation system **1** of the first embodiment). The wireless physical stimulation system comprises an electronic device, at least one sensor, and at least one physical stimulation device. The electronic device is wirelessly connected to the at least one sensor and at least one physical stimulation device.

Please refer to **FIG. 8**, the wireless physical stimulation method **8** may comprise the following steps of: in step **801**, detecting, by the at least one sensor, at least one first sensing signal; in step **803**, receiving, by the electronic device, the at least one first sensing signal from the at least one sensor; in step **805**, generating, by the electronic device, first body condition information on the basis of a body condition identifying model and according to the at least one first sensing signal, in step **807**, generating, by the electronic device, first feedback plan information on the basis of a first feedback model and according to the first body condition information; and in step **809**, controlling, by the electronic device, at least one physical stimulation device according to the first feedback plan information.

In one or some implementations, the wireless physical stimulation method **8** further comprises the step of: controlling, by the electronic device, a detection period of the at least one sensor and a feedback period of the at least one physical stimulation device, wherein the detection period and the feedback period do not overlap with each other. Other embodiments and technical descriptions are the same as those of the previous embodiments, and the detailed description is not repeated.

In one or some implementations, the physical stimulation device of the wireless physical stimulation system comprises both of a wireless thermotherapy device and a wireless electrotherapy device. The wireless physical stimulation method **8** further comprises the step of: controlling, by the electronic device, both of the wireless thermotherapy device and the wireless electrotherapy device according to the first feedback plan information.

In one or some implementations, the wireless physical stimulation method **8** further comprises the step of: receiving a user command via the electronic device, and adjusting the physical stimulation device via the electronic device according to the user command.

In one or some implementations, the wireless physical stimulation method **8** may be adapted for the wireless physical stimulation system **1**, and have the corresponding steps to achieve the functions of the wireless physical stimulation system **1.** Therefore, people skilled in the art may directly and comprehensively understand all of the corresponding steps of the wireless physical stimulation method **8** according to the aforesaid content of the wireless physical stimulation system **1**; thus, the content is not repeatedly described here.

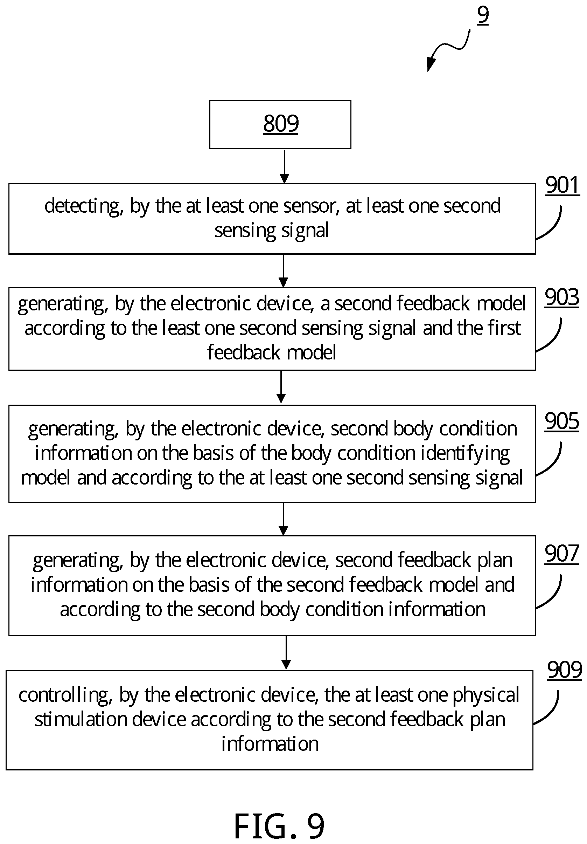

**FIG. 9** is a flow chart of a wireless physical stimulation method **9** of the ninth embodiment of the present invention. The wireless physical stimulation method **9** is an extension of the wireless physical stimulation method **8**; thus, the steps **801**, **803**, **805**, **807**, and **809** in **FIG.8** are not repeatedly described.

In addition to the steps **801**, **803**, **805**, **807**, and **809** in **FIG.8**, the wireless physical stimulation method **9** further comprises the steps of: in step **901**, detecting, by the at least one sensor, at least one second sensing signal; in step **903**, generating, by the electronic device, a second feedback model according to the least one second sensing signal and the first feedback model; in step **905**, generating, by the electronic device, second body condition information on the basis of the body condition identifying model and according to the at least one second sensing signal; in step **907**, generating, by the electronic device , second feedback plan information on the basis of the second feedback model and according to the second body condition information; and in step **909**, controlling, by the electronic device, the at least one physical stimulation device according to the second feedback plan information.

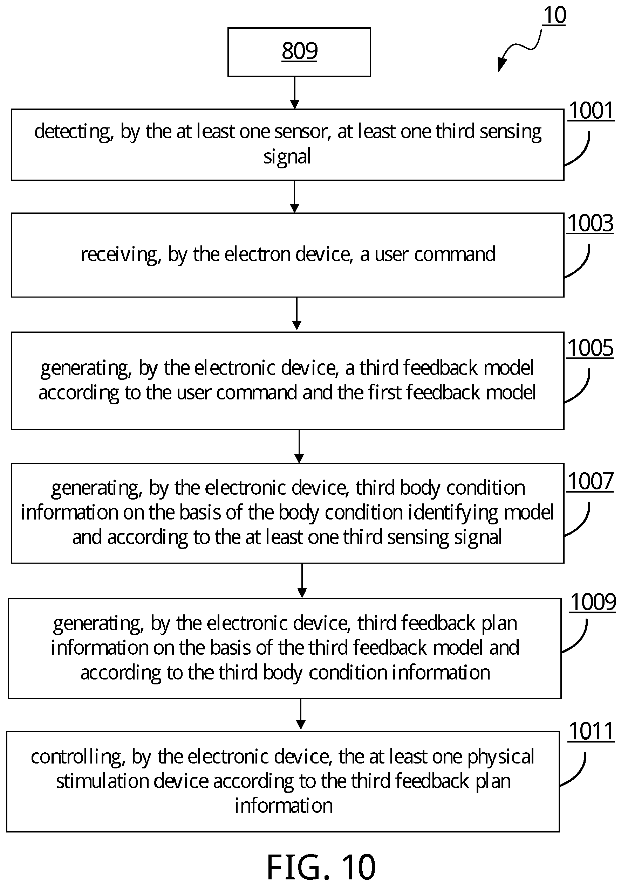

**FIG. 10** is a flow chart of a wireless physical stimulation method **10** of the tenth embodiment of the present invention. The wireless physical stimulation method **10** is an extension of the wireless physical stimulation method **8**; thus, the steps **801**, **803**, **805**, **807**, and **809** in **FIG.8** are not repeatedly described.

In addition to the steps **801**, **803**, **805**, **807**, and **809** in **FIG.8**, the wireless physical stimulation method **9** further comprises the steps of: in step **1001**, detecting, by the at least one sensor, at least one third sensing signal; in step **1003**, receiving, by the electron device, a user command; in step **1005**, generating, by the electronic device, a third feedback model according to the user command and the first feedback model; in step **1007**, generating, by the electronic device, third body condition information on the basis of the body condition identifying model and according to the at least one third sensing signal; in step **1009**, generating, by the electronic device, third feedback plan information on the basis of the third feedback model and according to the third body condition information, and in step **1011**, controlling, by the electronic device, the at least one physical stimulation device according to the second feedback plan information.

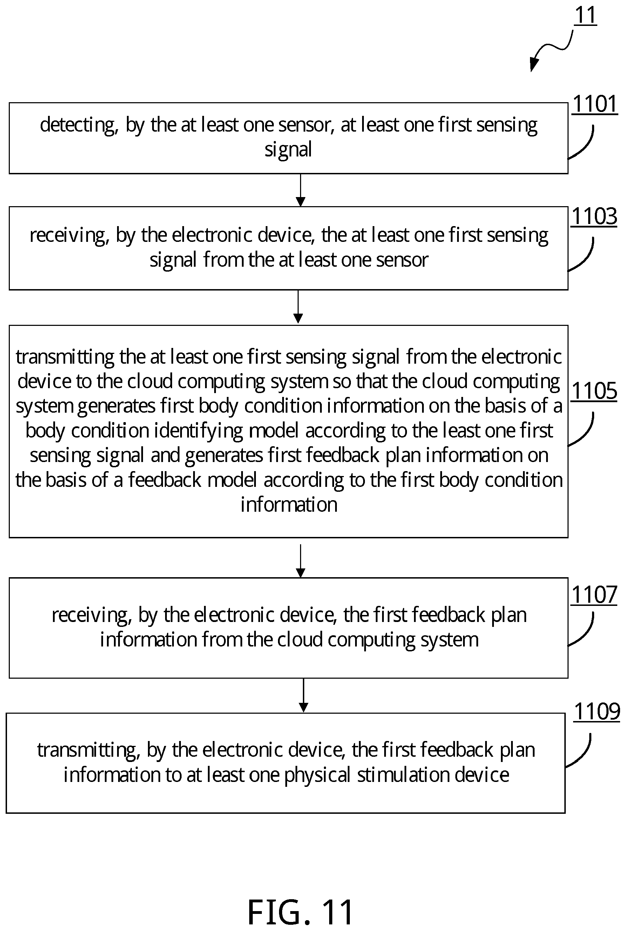

**FIG. 11** is a flow chart of a wireless physical stimulation method **11** of the eleventh embodiment of the present invention. The content shown in **FIG. 11** is merely used to describe the embodiment of the present invention rather than limit the present invention. The wireless physical stimulation method **11** is adapted for a wireless physical stimulation system (such as the wireless physical stimulation system **7** of the seventh embodiment). The wireless physical stimulation system comprises an electronic device, at least one sensor, and at least one physical stimulation device. The electronic device is wirelessly connected to a cloud computing system, the at least one sensor, and at least one physical stimulation device.

Please refer to **FIG. 11**, the wireless physical stimulation method **11** may comprise the following steps of: in step **1101**, detecting, by the at least one sensor, at least one first sensing signal; in step **1103**, receiving, by the electronic device, the at least one first sensing signal from the at least one sensor; in step **1105**, transmitting the at least one sensing signal from the electronic device to the cloud computing system so that the cloud computing system generates first body condition information on the basis of a body condition identifying model according to the least one first sensing signal, and generates first feedback plan information on the basis of a feedback model according to the first body condition information; in step **1107**, receiving, by the electronic device, the first feedback model from the cloud computing system; and in step **1109**, transmitting, by the electronic device, the first feedback plan information to at least one physical stimulation device.

In addition to the aforesaid eighth embodiment to the eleventh embodiment, the wireless physical stimulation method of the present invention is capable of performing all of the same functions mentioned in the aforesaid first embodiment to the seventh embodiment of the present invention, and delivers the same technical effects. Thus, the similar details are omitted. Besides, in the condition that the features do not conflict with each other, the aforesaid embodiments and implementations can be combined as an embodiment.

In summary, the wireless physical stimulation system and wireless physical stimulation method of the present invention determine body condition information according to a sensing signal of a sensor and a body condition identifying model and determine a physical stimulation feedback plan according to the body condition information and a feedback model. Therefore, in comparison with conventional thermotherapy/electrotherapy devices, the wireless physical stimulation system and the wireless physical stimulation method of the present invention further function as monitoring physiologic condition and providing a suitable physical stimulation plan in response to the physiologic condition of a user. Therefore, the present invention improves the drawbacks of the conventional thermotherapy/electrotherapy devices.

The above disclosure is related to the detailed technical contents and inventive features thereof. People of ordinary skill in the art may proceed with a variety of modifications and replacements based on the disclosures and suggestions of the invention as described without departing from the characteristics thereof. Nevertheless, although such modifications and replacements are not fully disclosed in the above descriptions, they have substantially been covered in the following claims as appended.

## Claims

1. A wireless physical stimulation system (1), comprising:
at least one sensor (13) disposed on a human body, being configured to detect at least one first sensing signal (S1);
at least one physical stimulation device (15) disposed on the human body; and
an electronic device (11), comprising:
a transceiver (111), wirelessly connected to the at least one sensor (13) and the at least one physical stimulation device (15) and being configured to receive the at least one first sensing signal (S1); and
a processor (113), electrically connected to the transceiver (111) and being configured to:
generate first body condition information (B1) on the basis of a body condition identifying model (M1) and according to the least one first sensing signal (S1);
generate first feedback plan information (C1) on the basis of a first feedback model (M1) and according to the first body condition information (B1); and
control, according to the first feedback plan information (C1), the at least one physical stimulation device (15).

2. The wireless physical stimulation system (2) of Claim 1, wherein the at least one sensor (13) is further configured to detect at least one second sensing signal (S2), and the processor (113) is further configured to:
establish, according to the at least one second sensing signal (S2) and the first feedback model (F1), a second feedback model (F2);
generate second body condition information (B2) on the basis of the body condition identifying model (M1) and according to the least one second sensing signal (S2);
generate second feedback plan information (C2) on the basis of the second feedback model (F2) and according to the second body condition information (B2); and
control, according to the second feedback plan information (C2), the at least one physical stimulation device (15).

3. The wireless physical stimulation (3) system of Claim 1, wherein:
the electronic device (11) further comprises an input unit (117) which is connected to the processor (113) and configured to receive a user command (U1);
the at least one sensor (13) is further configured to detect at least one third sensing signal (S3); and
the processor (113) is further configured to:
establish, according to the user command (Ul) and the first feedback model (F1), a third feedback model (F3);
generate third body condition information (B3) on the basis of the body condition identifying model (M1) and according to the at least one third sensing signal (S3);
generate third feedback plan information (C3) on the basis of the third feedback model (F3) and according to the third body condition information (B3); and
control, according to the third feedback plan information (C3), the at least one physical stimulation device (15).

4. The wireless physical stimulation system (5) of any of Claims 1 to 3, wherein:
the body condition identifying model (M1) further comprises a fatigue characteristic curve (V1) and a characteristic threshold (T1), wherein the fatigue characteristic curve (V1) is established by its characteristic data obtained by time-domain analysis and frequency-domain analysis to the at least one first sensing signal at a training stage, and the first body condition information (B1) further includes muscle fatigue condition information (E1);
the at least one sensor (13) comprises at least one electromyogram (EMG) sensor (13a), and the at least one first sensing signal (S1) comprises at least one EMG signal (S1a); and
the processor (113) is further configured to generate the muscle fatigue condition information (E1) on the basis of the fatigue characteristic curve (V1) and the characteristic threshold (T1) of the body condition identifying model (M1) according to the at least one EMG signal (Sla).

5. The wireless physical stimulation system (6) of any of Claims 1 to 3, wherein:
the body condition identifying model (M1) comprises a motion posture template (W1) and a characteristic threshold (XI), and the first body condition information (B1) comprises muscle fatigue condition information (E2);
the at least one sensor (13) comprises at least one motion sensor (13b) and the at least one first sensing signal (S1) comprises at least one motion signal (S1b); and
the processor (113) is further configured to generate, according to the at least one motion signal (S1b), the muscle fatigue condition information (E2) based on the motion posture template (W1) and the characteristic threshold (XI) of the body condition identifying model (M1).

6. The wireless physical stimulation system (1) of any of Claims 1 to 5, wherein the physical stimulation device (15) is one of a wireless thermotherapy device and a wireless electrotherapy device, and the processor (113) is further configured to control one of the wireless thermotherapy device and the wireless electrotherapy device according to the first feedback plan information (C1).

7. The wireless physical stimulation system (1) of any of Claims 1 to 6, wherein the electronic device (11) further comprises an input unit, the input unit is electrically connected to the processor (113)and is configured to receive a user command, and the processor (113) is further configured to adjust the at least one physical stimulation device (15) according to the user command.

8. A wireless physical stimulation system (7), comprising:
at least one sensor (13) disposed on a human body, being configured to detect at least one first sensing signal (S1);
at least one physical stimulation device (15) disposed on the human body; and
an electronic device (11), comprising:
a transceiver (111), wirelessly connected to a cloud computing system (73), the at least one sensor (13) and the at least one physical stimulation device (15) and being configured to:
receive the at least one first sensing signal (S1);
transmit the at least one first sensing signal (S1) to the cloud computing system (73) so that the cloud computing system (73) generates first body condition information (B1) on the basis of a body condition identifying model (M1) and according to the at least one first sensing signal (S1) and generates first feedback plan information (C1) on the basis of a feedback model (F1) and according to the first body condition information (B1);
receive the first feedback plan information (C1) from the cloud computing system (73); and
transmit the first feedback plan information (C1) to the at least one physical stimulation device (15).

9. A wireless physical stimulation method (8), being adapted for a wireless physical stimulation system (1) which includes at least one sensor (13), at least one physical stimulation device (15) and an electronic device (11), the at least one sensor (13) and the at least one physical stimulation device (15) being disposed on a human body, the electronic device (11) being wirelessly connected to the at least one sensor (13) and the at least one physical stimulation device (15), the wireless physical stimulation method (8) comprising the following steps of:
detecting, by the at least one sensor (13), at least one first sensing signal (S1);
receiving, by the electronic device (11), the at least one first sensing signal (S1) from the at least one sensor (13);
generating, by the electronic device (11), first body condition information (B1) on the basis of a body condition identifying model (M1) and according to the at least one first sensing signal (S1);
generating, by the electronic device (11), first feedback plan information (C1) on the basis of the first feedback model (F1) and according to the first body condition information (B1); and
controlling, by the electronic device (11), at least one physical stimulation device (15) according to the first feedback plan information (C1).

10. The wireless physical stimulation method (9) of Claim 9, further comprising the following steps of:
detecting, by the at least one sensor (13), at least one second sensing signal (S2);
generating, by the electronic device (11), a second feedback model (F2) according to the least one second sensing signal (S2) and the first feedback model (F1);
generating, by the electronic device (11), second body condition information (B2) on the basis of the body condition identifying model (M1) and according to the at least one second sensing signal (S2);
generating, by the electronic device (11), second feedback plan information (C2) on the basis of the second feedback model (F2) and according to the second body condition information (B2); and
controlling, by the electronic device (11), the at least one physical stimulation device (15) according to the second feedback plan information (C2).

11. The wireless physical stimulation method (10) of Claim 9, further comprising the following steps of:
detecting, by the at least one sensor (13), at least one third sensing signal (S3);
receiving, by the electron device (11), a user command (U1);
generating, by the electronic device (11), a third feedback model (F3) according to the user command (Ul) and the first feedback model (F1);
generating, by the electronic device (11), third body condition information (B3) on the basis of the body condition identifying model (M1) and according to the at least one third sensing signal (S3);
generating, by the electronic device (11), third feedback plan information (C3) on the basis of the third feedback model (F3) and according to the third body condition information (B3); and
controlling, by the electronic device (11), the at least one physical stimulation device (15) according to the third feedback plan information (C3).

12. The wireless physical stimulation method (8) of any of Claims 9 to 11, wherein the body condition identifying model (M1) further comprises a fatigue characteristic curve (VI) and a characteristic threshold (Tl), wherein the fatigue characteristic curve (VI) is established by its characteristic data obtained by time-domain analysis and frequency-domain analysis to the at least one first sensing signal (S1) at a training stage, and the first body condition information (B1) includes muscle fatigue condition information (El), the at least one sensor (13) comprises at least one EMG sensor (13a), the at least one first sensing signal (S1) comprises at least one EMG signal (Sla), and the wireless physical stimulation method (8) further comprises the following step of:
generating, by the electronic device (11), the muscle fatigue condition information (E1) on the basis of the fatigue characteristic curve (V1) and the characteristic threshold (Tl) of the body condition identifying model (M1) and according to the at least one EMG signal (Sla).

13. The wireless physical stimulation method (8) of any of Claims 9 to 11, wherein the body condition identifying model (M1) comprises a motion posture template (W1) and a characteristic threshold (X1), and the first body condition information (B1) comprises muscle fatigue condition information (E2);
the at least one sensor (13) comprises at least one motion sensor (13b) and the at least one first sensing signal (S1) comprises at least one motion signal (S1b); and
the wireless physical stimulation method (8) further comprises the following step:
generating, by the electronic device (11), the muscle fatigue condition information (E2) on the basis of the motion posture template (W1) and the characteristic threshold (X1) of the body condition identifying model (M1) and according to the at least one motion signal (S1b).

14. The wireless physical stimulation method (8) of any of Claims 9 to 13, wherein the physical stimulation device (15) is one of a wireless thermotherapy device and a wireless electrotherapy device, and the wireless physical stimulation method (8) further comprises the following step of:
controlling, by the processor (113), one of the wireless thermotherapy device and the wireless electrotherapy device according to the first feedback plan information (C1).

15. The wireless physical stimulation method (8) of any of Claims 9 to 14, wherein comprising: receiving, by the electronic device (11), a user command, and adjusting, by the processor (113), the physical stimulation device (15) according to the user command.
